# EUROPÄISCHE PATENTANMELDUNG

(11) **EP 2 098 196 A1**
(43) Veröffentlichungstag der Anmeldung: **09.09.2009**
(21) Anmeldenummer: 09003170.9
(22) Anmeldetag: 05.03.2009
(51) Int. Cl.: A61F 5/00

(54) **Fixiervorrichtung für Knochenbrüche**

(30) Priorität: 05.03.2008 DE 102008012709
(71) Anmelder: Huber, Karin, 7000 Chur (CH)
(72) Erfinder: Huber, Karin, 7000 Chur (CH)
(74) Vertreter: Goy, Wolfgang

(57) **Zusammenfassung**

Die Erfindung betrifft eine Vorrichtung zum Fixieren eines gebrochenen Knochens 1 des menschlichen oder tierischen Körpers zum Wiederzusammenwachsen des Bruches 2. Die Fixiereinrichtung ist durch eine Manschette 5 mit einem Hohlraum 6 gebildet, welcher mit einem Medium, insbesondere Luft füllbar ist.

## Beschreibung

Die Erfindung betrifft eine Vorrichtung zum Fixieren eines gebrochenen Knochens des menschlichen oder tierischen Körpers zum Wiederzusammenwachsen des Knochens nach dem Oberbegriff des Anspruchs 1.

Wenn ein Mensch oder ein Tier sich einen Knochen gebrochen hat, muß dieser fixiert werden, damit er wieder zusammenwachsen kann. Hierzu gibt es bislang drei Verfahren:
Erstens kann das Körperteil (insbesondere die Gliedmaßen) vollümfänglich eingegipst werden. Da ein derartiger Gips relativ schwer ist, ist der Mensch dadurch in den täglichen Bewegungsabläufen sehr gehandicapt. Außerdem besteht beim Gips das Problem darin, daß er nicht wasserbeständig ist, d. h. er löst sich im Kontakt mit Wasser allmählich auf. Ein Duschen ist somit nur dann möglich, wenn der Gips vor Wasser geschützt wird.
Zweitens ist es bekannt, das Körperteil im Bereich des Bruches mit Binden zu bandagieren. Da dies jedoch für die Fixierung des Knochens nicht ausreicht, ist es zusätzlich erforderlich, das Körperteil in einer Kunststoffschale zu fixieren. Auch hier besteht der Nachteil darin, daß die Bewegungsfreiheit erheblich eingeschränkt ist. Außerdem ist es auch hier nicht möglich, daß die Person duschen kann. Denn der Patient muß unter die Kunststoffschale eine Art dicken elastischen Strumpf anziehen, um Druckstellen zu vermeiden.

Drittens ist es bekannt, den Knochen operativ unter Verwendung von eingeschraubten Platten zu fixieren. Hier handelt es sich um eine Operation mit den entsprechenden Risiken. Außerdem müssen nach einer gewissen Zeit die Platten sowie die Schrauben wieder entfernt werden, was eine erneute Operation bedeutet.

Davon ausgehend liegt der Erfindung daher die **Aufgabe** zugrunde, eine einfach handhabbare Vorrichtung zum Fixieren eines gebrochenen Knochens des menschlichen oder tierischen Körpers zum Wiederzusammenwachsens des Knochens zu schaffen.

Die technische **Lösung** ist gekennzeichnet durch die Merkmale im Kennzeichen des Anspruchs 1.

Dadurch ist eine Vorrichtung zum Fixieren eines gebrochenen Knochens des menschlichen oder tierischen Körpers zum Wiederzusammenwachsen des Knochens geschaffen, welche sehr einfach gehandhabt werden kann. Es handelt sich dabei um ein orthopädisches Fraktur Management System für die Nachbehandlung von Frakturen. Die erfindungsgemäße Manschette ermöglicht die Behandlung von Frakturen jeglicher Art, vor allem jener, die nicht operativ, also mit eingeschraubten Platten behandelt werden können. Die erfindungsgemäße Manschette dient zur Ruhigstellung der Fraktur und schützt die Fraktur bei Mensch und Tier. Dabei sind Brüche an Unterarm, Oberarm, Schulter, Oberkörper, Beine etc. ohne weiteres behandelbar. Selbstverständlich ist die Größe der Manschette an das jeweilige Körperteil sowie an die Form und Art des Bruches angepaßt. Der Vorteil einer derartigen Manschette besteht darin, daß nirgends Druckstellen vorhanden sind, wie dies bei den bisher üblichen Gips- und Plastikformen der Fall ist. Die Manschette stützt die Frakturstelle nachhaltig, stellt die betroffenen Körperteile ruhig, behindert jedoch Mensch und Tier nicht in der Bewegung. Die Manschette ist nachhaltig, wiederverwendbar, einfach in der Handhabung und fördert die Mobilität des Betroffenen. Auch ist es ohne weiteres leicht möglich, für Zwischenuntersuchungen die Manschette zu entfernen.

Um die Manschette zu füllen, kann gemäß der Weiterbildung in Anspruch 2 Luft oder ein anderes geeignetes Gas verwendet werden. Es ist daher lediglich erforderlich, die Luft in die Manschette einzublasen. Dies kann mittels eines entsprechenden Pumpaggregats erfolgen. Dies erfolgt vorzugsweise über ein Rückschlagventil, welches gleichermaßen auch geöffnet werden kann, damit die Luft wieder entweichen kann. Insbesondere ist es möglich, durch langsames Einströmen der Luft allmählich einen Druck auf das Körperteil und damit auf den Knochen ausüben zu können. Dadurch kann sogar eine Selbstausrichtung der gebrochenen beiden Teile des Knochens erreicht werden. Denn die Manschette hat den Vorteil, daß durch die allmähliche, allseitige Druckausübung eine Zentrierung des Knochens erfolgt.

Alternativ ist es gemäß Anspruch 3 auch möglich, daß der Hohlraum mittels eines viskosen Materials, insbesondere eines Gels befüllbar ist. Das viskose Material hat den Vorteil, daß es eine gewisse innere Stabilität aufweist und somit die Manschette sehr schnell eine stabile Form erhält. Auch hier ist das viskose Material mittels entsprechenden Pumpen einfüllbar.

Gemäß der Weiterbildung in Anspruch 4 ist es auch denkbar, als Füllmaterial ein ausschäumendes Material zu verwenden. Dies hat den Vorteil, daß es ähnlich wie die Luft sehr leicht ist, daß aber im ausgeschäumten Zustand ein stabiler Hohlraum geschaffen ist, welcher sich optimal der Körperteilkontur anpaßt.

Schließlich schlägt die Weiterbildung gemäß Anspruch 5 als Material ein aushärtendes Material vor. Dieses ist zwar etwas schwerer als die vorerwähnten Materialien, es besitzt jedoch eine sehr hohe Stabilität.

Die Weiterbildung gemäß Anspruch 6 schlägt vor, daß die Fixiervorrichtung aus einem wasserbeständigen Material besteht. Dies hat den Vorteil, daß trotz der Manschette die Person duschen kann. Vorzugsweise wird hautfreundliches Latex verwendet, welches sich in der Vergangenheit als Material bewährt hat. Es ist weiterhin denkbar, daß das Manschettenmaterial atmungsaktiv und luftdurchlässig ist, so daß die Haut atmen kann. Voraussetzung hierfür ist jedoch, daß das Material, welches sich im Hohlraum der Manschette befindet, nicht entweichen kann. Schließlich sind weitere Faktoren für das Manschettenmaterial Klimaorientiertheit, Robustheit, hohe Elastizität, guter Tragekomfort sowie Formstabilität. Es kann sich dabei um ein Doppelgewebe mit gutem Feuchtigkeitstransport handeln. Dabei kann außen Synthetik und innen eine Funktionsfaser vorgesehen sein. Ein derartiges Gewebe ist rasch trocknend und hoch atmungsaktiv, leicht, strapazierfähig und dauerelastisch und gewährt darüber hinaus eine enorme Bewegungsfreiheit.

In einer ersten Ausführungsform wird gemäß der Weiterbildung in Anspruch 7 vorgeschlagen, daß die Manschette schlauchförmig ausgebildet ist. Dies bedeutet, daß die Manschette im leeren Zustand über den Arm oder das Bein im Bereich des Bruches gestülpt wird, um anschließend dann den Hohlraum mit dem Medium zu füllen. Der Vorteil dieser schlauchförmigen Gestaltung der Manschette besteht darin, daß sie allseitig um den Arm oder das Bein anliegt und somit einen gleichmäßigen, radial nach innen gerichteten Druck ausübt.

Alternativ kann gemäß der Weiterbildung in Anspruch 8 die Manschette auch ein streifenförmiges Gebilde sein, welches um das Körperteil gewickelt und dann fixiert wird. Dies bedeutet, daß die Manschette in der Art einer Manschette für ein Blutdruckmeßgerät aufgebaut ist. Die Fixierung in der Wickelform erfolgt vorzugsweise mittels eines Klettverschlusses.

Für Rüppenbrüche und Schulterarmbrüche kann die Manschette auch gemäß der Weiterbildung in Anspruch 9 in Form eines Jäckchens ausgebildet sein, das man anzieht und das durch Luft stabil bleibt und die gebrochene Körperstelle schützt und stützt.

Eine weitere bevorzugte Weiterbildung schlägt gemäß Anspruch 10 vor, daß um die Manschette herum eine starre Schale angeordnet ist, an deren Innenwand sich die Manschette im befüllten Zustand abstützt. Bei dieser starren Schale handelt es sich vorzugsweise um eine Kunststoffschale. Hier ist es von Vorteil, wenn diese Kunststoffschale an die Kontur des Körperteils angepaßt ist. Denn dies hat den Vorteil, daß beim Befüllen der Manschette eine gleichmäßige, radial nach innen gerichtete Druckausübung bewirkt wird. Diese Schale wird gleichermaßen wie die vorzugsweise schlauchförmige Manschette über den Arm oder das Bein gestülpt. Um die gegenseitige Positionierung zwischen der inneren Manschette und der äußeren Schale zu verbessern, kann die Manschette mit ihrer Außenwandung an der Innenwandung der Schale befestigt sein, so daß die äußere Schale mit der inneren Manschette eine bauliche Einheit bildet.

Die Weiterbildung gemäß Anspruch 11 geht von dem Grundgedanken aus, daß durch das Befüllen der Manschette mit dem Medium derart beidseits des Bruches eine entgegengesetzte Kraft auf die beiden Knochenteile ausgeübt wird, daß diese in Axialrichtung zusammengepreßt werden. Dies hat den Vorteil, daß während des Befüllens der Manschette mit dem Medium die Knochen in die gewünschte Position gebracht werden, also auch in Axialrichtung des Knochens.

Eine weitere bevorzugte Weiterbildung schlägt gemäß Anspruch 12 eine spezielle Profilierung der Manschette vor. Der Grundgedanke ist dabei Folgender: Die Körperoberfläche ist grundsätzlich unregelmäßig, bedingt durch den Muskelaufbau. Dies bedeutet aber, daß beim Befüllen der Manschette mit dem Medium sich zunächst ungleiche Druckverteilungen ergeben. Wenn aber die Innenseite der Manschette derart strukturiert und/oder profiliert ausgebildet ist, daß bei Abstandsvergrößerungen zwischen der Manschette und der Körperoberfläche in diesem Bereich die Manschette mehr gedehnt wird, so wird dort ein gleich großer Druck aufgebaut wie in den restlichen Bereichen der Körperoberfläche. Dadurch wird eine sehr gleichmäßige, radial nach innen gerichtete Kraft auf den Knochen ausgeübt, so daß dieser eine exakt vorgegebene Position einhält.

Schließlich schlägt die Weiterbildung gemäß Anspruch 13 eine Befestigungsvorrichtung für die Manschette am Oberkörper der Person vor. Beispielsweise kann bei Armbrüchen zusätzlich eine überlappende, dünne Gummistoffhaut über die Manschette gezogen werden, die mit einem um den Körper gelegten Band verbunden ist, um den Arm in einer bestimmten Lage zu halten. Selbstverständlich ist diese Befestigungsvorrichtung ebenfalls wasserdicht und luftdurchlässig.

Zwei Ausführungsbeispiele einer erfindungsgemäßen Vorrichtung zum Fixieren eines gebrochenen Knochens des menschlichen oder tierischen Körpers zum Wiederzusammenwachsen des Knochens wird nachfolgend anhand der Zeichnungen beschrieben. In diesen zeigt:
- Fig. 1a: eine schematische Längsschnittdarstellung im Bereich eines gebrochenen Oberarmes vor dem Befüllen der Manschette;
- Fig. 1b: eine schematische Längsschnittdarstellung im Bereich eines gebrochenen Oberarmes nach dem Befüllen der Manschette;
- Fig. 2: eine schematische Darstellung bei einem Rippenbruch.

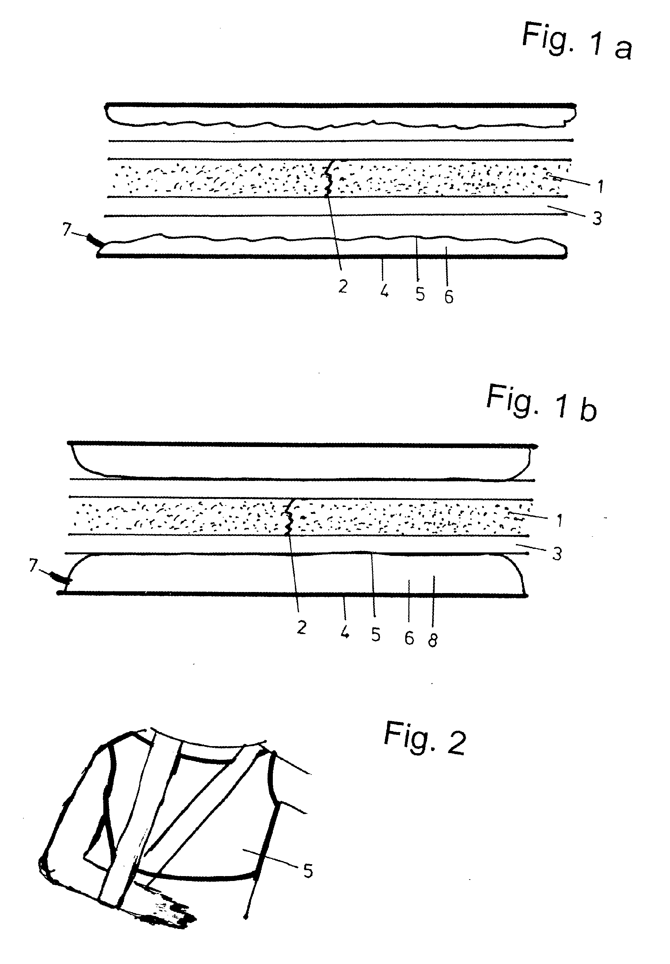

In Fig. 1 ist der gebrochene Knochen 1 eines Oberarms schematisch dargestellt. Der Bruch 2 ist dabei in diesem Ausführungsbeispiel relativ glatt. Selbstverständlich kann der Bruch 2 auch komplizierter ausgebildet sein, beispielsweise spiralförmig.

Um den Bruch 2 des Knochens 1 zu fixieren, wird das entsprechende Körperteil 3 mit einer Fixiervorrichtung umgeben. Diese Fixiervorrichtung besteht zunächst aus einer rohrförmigen Schale 4 aus Kunststoff. An der Innenseite dieser Schale 4 ist eine schlauchförmige Manschette 5 befestigt, so daß die Schale 4 und die Manschette 5 eine bauliche Einheit bilden. Die Manschette 5 besitzt einen Hohlraum 6 sowie ein Ventil 7 für die Zuführung eines Mediums.

Die Funktionsweise ist wie folgt:
Die Einheit aus Schale 4 und Manschette 5 wird über das gebrochene Körperteil 3 geschoben, im vorliegenden Beispiel über den Oberarm. Dabei ist der Hohlraum 6 der Manschette 5 noch nicht mit dem Medium gefüllt (Fig. 1a).

Nachdem die Manschette 5 ihre Position erreicht hat, wird über das Ventil 7 der Hohlraum 6 mit dem Medium 8 befüllt (Fig. 1b). Bei diesem Medium 8 kann es sich um Luft, um ein Gel, um ein ausschäumendes Material oder ein aushärtendes Material handeln. Vorzugsweise wird Luft verwendet.

Durch allmähliches Aufpumpen des Hohlraums 6 mit der Luft legt sich die Manschette an dem Körperteil 3 an und übt radial nach innen gerichtet einen gleichmäßigen Druck auf das Körperteil 3 und damit auf den Knochen 1 aus. Dabei werden die beiden Teile des Knochens 1 derart zusammengepreßt, daß die Bruchstellen aneinanderliegen. Der Druck in dem Hohlraum 6 der Manschette 5 ist dabei derart zu erhöhen, daß eine dauerhafte und nachhaltige Fixierung des Knochens 1 erreicht wird.

Nach Heilung des Bruches 2 kann die Manschette 5 mit ihrer Schale 4 wieder leicht entfernt werden, indem einfach die Luft über das Ventil 7 abgelassen wird.

Ein weiteres Ausführungsbeispiel in Fig. 2 zeigt einen Rippenbruch oder einen Schulterarmbruch. Hier ist die Manschette 5 nicht schlauchförmig wie beim Oberarmbruch ausgebildet, sondern in Form eines Jäckchens, welches die Person anzieht. Die Funktionsweise ist dabei die gleiche wie beim Oberarmbruch, d. h. die angezogene Manschette 5 in Form des Jäckchens wird aufgepumpt, um den Rippenbruch oder Schulterarmbruch stabil zu halten und die Körperstelle zu stützen und zu schützen. Auch in diesem Fall ist es zur Stützung der jäckchenförmigen Manschette 5 denkbar, diese von einer Schale 4 aus Kunststoff zu umgeben, welcher der Außenmantelung der Manschette 5 mehr Stabilität verleiht.

In diesem Fall ist der Arm mittels einer Schleife, insbesondere mittels eines luftdurchlässigen Bandes am Körper fixiert.

### Bezugszeichenliste

- 1: Knochen
- 2: Bruch
- 3: Körperteil
- 4: Schale
- 5: Manschette
- 6: Hohlraum
- 7: Ventil
- 8: Medium

## Patentansprüche

1. Vorrichtung zum Fixieren eines gebrochenen Knochens (1) des menschlichen oder tierischen Körpers zum Wiederzusammenwachsen des Knochens (1),
wobei die Fixierung das Körperteil im Bereich des Bruches (2) vollumfänglich umschließt,
**dadurch gekennzeichnet,**
**daß** die Fixiervorrichtung durch eine, einen geschlossenen Hohlraum (6) aufweisende, zumindest in Teilbereichen flexible Manschette (5) gebildet ist,
wobei der Hohlraum (6) der Manschette (5) mit einem Medium (8) derart befüllbar ist, daß sich die Manschette (5) aufbläht und **dadurch** am Körperteil zum Anliegen kommt.

2. Vorrichtung nach Anspruch 1,
**dadurch gekennzeichnet,**
**daß** der Hohlraum (6) mit Luft oder einem anderen Gas füllbar ist.

3. Vorrichtung nach Anspruch 1,
**dadurch gekennzeichnet,**
**daß** der Hohlraum (6) mittels eines viskosen Materials, insbesondere einem Gel füllbar ist.

4. Vorrichtung nach Anspruch 1,
**dadurch gekennzeichnet,**
**daß** der Hohlraum (6) mittels eines aufschäumenden Materials füllbar ist.

5. Vorrichtung nach Anspruch 1,
**dadurch gekennzeichnet,**
**daß** der Hohlraum (6) mittels eines aushärtenden Materials füllbar ist.

6. Vorrichtung nach einem der vorhergehenden Ansprüche,
**dadurch gekennzeichnet,**
**daß** die Fixiervorrichtung aus einem wasserbeständigen Material besteht.

7. Vorrichtung nach einem der Ansprüche 1 bis 6,
**dadurch gekennzeichnet,**
**daß** die Manschette (5) schlauchförmig ausgebildet ist.

8. Vorrichtung nach einem der Ansprüche 1 bis 6,
**dadurch gekennzeichnet,**
**daß** die Manschette (5) ein streifenförmiges Gebilde ist, welche um das Körperteil wikkelbar und in der Wickelposition fixierbar ist.

9. Vorrichtung nach einem der Ansprüche 1 bis 6,
**dadurch gekennzeichnet,**
**daß** die Manschette (5) jackenförmig ausgebildet ist.

10. Vorrichtung nach einem der vorhergehenden Ansprüche,
**dadurch gekennzeichnet,**
**daß** um die Manschette (5) herum eine starre Schale (4) angeordnet ist, an deren Innenwand sich die Manschette (5) im befüllten Zustand abstützt.

11. Vorrichtung nach einem der vorhergehenden Ansprüche,
**dadurch gekennzeichnet,**
**daß** die Manschette (5) derart profiliert ist, daß sie im befüllten Zustand entgegengesetzt gerichtete Kräfte auf den Knochen (1) ausübt, der diesen im Bereich des Bruches (2) zusammenpreßt.

12. Vorrichtung nach einem der vorhergehenden Ansprüche,
**dadurch gekennzeichnet,**
**daß** die Manschette (5) auf der der Körperoberfläche zugewandten Innenseite in der Fläche derart strukturiert und/oder profiliert ist, daß sie entsprechend der Oberflächenkontur des umschließenden Körperteils mit gleichmäßigem Druck anliegt.

13. Vorrichtung nach einem der vorhergehenden Ansprüche,
**dadurch gekennzeichnet,**
**daß** die Manschette (5) mit einer Befestigungsvorrichtung zum Fixieren der Manschette (5) am Oberkörper vorgesehen ist.
